# FASCICULE DE BREVET EUROPEEN

(11) **EP 0 442 807 B1**
(45) Date de publication et mention de la délivrance du brevet: **13.05.1998**
(21) Numéro de dépôt: 91400356.1
(22) Date de dépôt: 13.02.1991
(51) Int. Cl.: A61N 1/375

(54) **Connecteur mâle universel pour sonde monopolaire de stimulation cardiaque**
Universaler Steckverbinder für monopolare Herzreizungssonde
Universal male connector for monopolar cardiac stimulation probe

(30) Priorité: 14.02.1990 FR 9001728
(43) Date de publication de la demande: 21.08.1991
(73) Titulaire: ELA MEDICAL, F-92541 Montrouge (FR)
(72) Inventeur: Olivier, Stéphane, F-45770 Saran (FR)
(74) Mandataire: Loyer, Bertrand

(56) Documents cités:
- EP-A- 343 402
- DE-A- 2 739 270
- FR-A- 2 504 383
- FR-A- 2 568 424
- GB-A- 612 213
- US-A- 3 157 455

## Description

La présente invention concerne d'une manière générale les stimulateurs cardiaques et de manière plus précise les moyens de connexion utilisés entre la sonde cardiaque et le stimulateur.

A l'heure actuelle, pratiquement chaque fabricant de stimulateurs cardiaques réalise la connexion entre les stimulateurs et les sondes qu'il fabrique par des moyens qui lui sont propres. Ceci oblige à toujours remplacer un stimulateur par un stimulateur de même type.

Afin de permettre l'adaptation de tout stimulateur à toutes les sondes, les milieux professionnels mettent actuellement au point une norme internationale définissant les connecteurs mâles et femelles qui devront dans un proche avenir être utilisés sur tous les modèles de stimulateurs cardiaques implantables. Cette norme est actuellement connue sous forme de projet sous la référence "Entwurf-DIN-VDE 0750" (Novembre 1988) et un connecteur mâle conforme à cette norme est représenté à la Fig. 1. Le document EP 0 343 402 décrit un connecteur pour sonde bipolaire, dont les caractéristiques extérieures satisfont à cette norme.

Il se pose alors le problème de réaliser la connexion de stimulateurs nouveaux aux normes avec une sonde ancienne déjà implantée chez un patient. En effet, lorsque l'on change un stimulateur afin de toujours faire bénéficier le patient des derniers progrès de la technique, on change très rarement la sonde car celle-ci a une durée de vie plus grande que le stimulateur et de plus est souvent difficile à extraire.

Il a été proposé des prolongateurs portant à une extrémité un connecteur mâle normalisé et à l'autre extrémité un connecteur femelle s'adaptant au connecteur mâle de la sonde en place. Ces dispositifs sont encombrants et donc peu faciles à implanter car une longueur non négligeable est nécessairement prévue entre les deux connecteurs afin de faciliter les manipulations. De plus, le clinicien doit être en possession d'autant de prolongateurs qu'il existe de types de connexion sur le marché.

Il a également été proposé des adaptateurs se voulant être plus universels mais présentant d'autres inconvénients. Certains ne sont pas totalement universels, d'autres posent des problèmes d'étanchéité ou de manipulation, d'autres encore ne sont plus conformes à la norme après fixation avec le conducteur. Tous ces modèles sont utilisés après avoir coupé le connecteur de la sonde en place.

A titre d'exemple, le document DE-A-3 306 115 décrit un connecteur mâle universel de remplacement monté sur une sonde cardiaque monopolaire formée par une gaine dans laquelle est positionné un fil conducteur enroulé en spirale, qui est formé par deux parties solidaires l'une de l'autre, la première partie étant formé d'un manchon arrière en matière plastique présentant des anneaux et traversé par un alésage dans lequel peut coulisser le fil conducteur de la sonde, la deuxième partie étant formée d'une fiche distale électriquement conductrice sur l'axe de laquelle se place le fil conducteur de la sonde, une collerette fendue assurant la liaison mécanique entre la fiche distale et le manchon arrière.

La présente invention a alors pour but de proposer un connecteur mâle universel correspondant aux normes, qui puisse être facilement mis en place à l'extrémité de toute sonde monopolaire en remplacement du connecteur mâle de celle-ci, garantissant une liaison mécanique et électrique fiable ainsi qu'une parfaite étanchéité.

A cet effet le connecteur mâle universel de remplacement d'un ancien connecteur supprimé, de dimensions normalisées, destiné à être monté sur une sonde cardiaque monopolaire formée par une gaine dans laquelle est positionné un fil conducteur enroulé en spirale, qui est formé par deux parties solidarisables l'une à l'autre,
- la première partie étant formée d'un manchon arrière en matière plastique présentant des anneaux et traversé par un alésage dans lequel peut coulisser le fil conducteur de la sonde,
- la deuxième partie étant formée d'une fiche distale électriquement conductrice
- une bague proximale rigide dépassant dudit manchon du côté des anneaux , s'étendant dans la première partie, à l'intérieur du manchon arrière, sur au moins une partie de celui-ci,
- la fiche distale portant dans la deuxième partie, un manchon avant en matière plastique globalement au milieu de sa longueur ; ledit manchon avant présentant des anneaux et la fiche distale conductrice dépassant dudit manchon du côté des anneaux ;
- la bague proximale de la première partie étant traversée par un alésage dans lequel peut coulisser le fil conducteur de la sonde,
   est caractérisée en ce que :
   un moyen conducteur présentant une certaine élasticité et ayant globalement la forme d'un Z couché est prévu en combinaison avec la bague proximale afin d'assurer une liaison électrique entre le fil conducteur et la fiche distale ainsi qu'une liaison mécanique entre le fil conducteur (2) et le connecteur formé des premier (3) et deuxième partie (4) et en ce que l'alésage est taraudé tandis que l'extrémité de la pièce conductrice portant ledit moyen conducteur est filetée.

Le connecteur est également caractérisé en ce que :
- le moyen conducteur présentant une certaine élasticité est un stylet solidaire de la deuxième partie, formé par une pièce conductrice de diamètre externe inférieur au diamètre interne de l'enroulement du fil conducteur, ledit stylet se terminant par des portions axiales, la distance séparant les deux portions radialement externes étant supérieure au diamètre de l'alésage de la bague proximale de la première partie ;
- l'alésage de la première partie est formé d'une portion dans le manchon arrière et dans la bague, de diamètre supérieur au maximum des diamètres externes des conducteurs existants, raccordée par une portion conique à un alésage de plus grand diamètre ;
- le manchon arrière est recouvert d'une jupe souple élastique au cours du montage destinée à être collée sur la face externe de la gaine ;
- l'alésage prévu dans la bague proximale peut être strié annulairement ;
- la jupe possède un étranglement qui comprime la gaine.

La présente invention sera mieux comprise grâce à la description qui va suivre d'un mode de réalisation de celle-ci en référence aux figures sur lesquelles :
La figure 1 représente le connecteur mâle normalisé ;
Les figures 2 à 9 représentent les différentes étapes du montage de l'adaptateur selon l'invention.

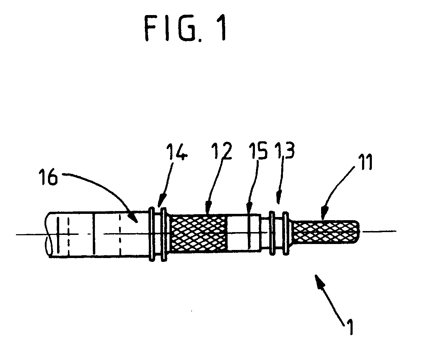

On voit à la figure 1 le connecteur mâle tel qu'il est préconisé par la norme. Ce connecteur comporte deux zones électriquement conductrices 11, 12 de diamètres différents dans le cas d'une sonde bipolaire. Une seule zone électriquement conductrice 11 est utile dans le cas d'une sonde monopolaire. Deux zones 13, 14 comportant des anneaux, de diamètre plus grand que les zones conductrices, destinés à s'appliquer contre la paroi interne du connecteur femelle prévu sur le stimulateur, sont agencées afin d'assurer l'étanchéité de la connexion et éviter le glissement relatif intempestif du connecteur mâle dans le connecteur femelle.

Deux zones non conductrices 15, 16 sont de plus prévues l'une entre la première zone 13 comportant des anneaux et la seconde zone 12 et l'autre à l'arrière du connecteur à côté de la zone 14. Ces zones 15, 16 sont destinées à réaliser l'étanchéité avec d'éventuels anneaux d'étanchéité pouvant être prévus à l'intérieur du connecteur femelle du stimulateur.

La norme définit les diamètres et longueurs de chacune des zones du connecteur ; elle propose aussi des tests mécaniques et électriques permettant de garantir une étanchéité maximale tout en minimisant les efforts de connexion.

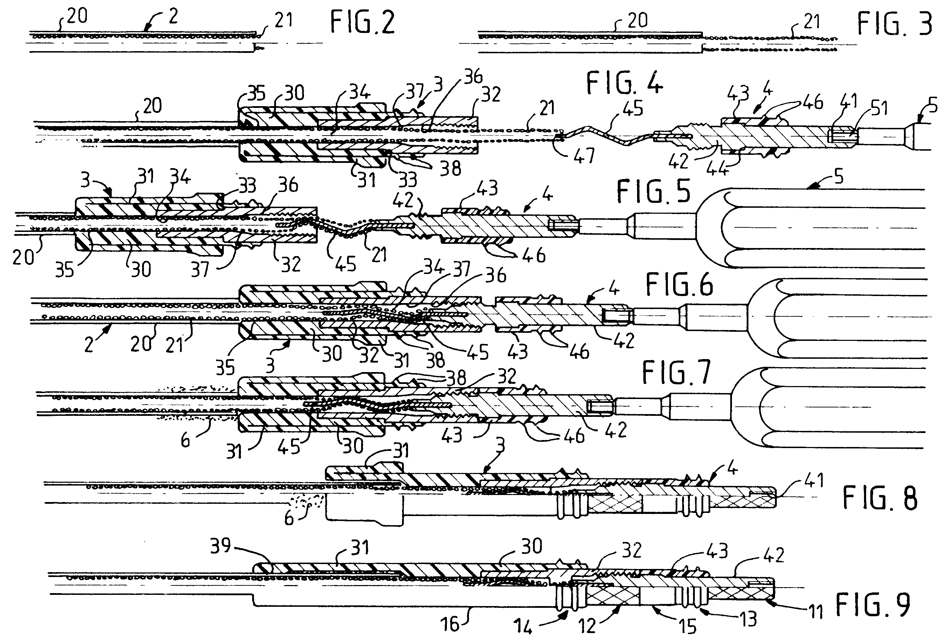

Nous allons maintenant décrire le connecteur selon l'invention en même temps que son mode de montage en référence aux figures 2 à 9. A la figure 2, on voit l'extrémité d'une sonde 2 cardiaque que l'on a coupé afin d'enlever le connecteur non normalisé qu'elle portait. De manière connue, une telle sonde est formée par une gaine 20 en une matière plastique compatible avec son implantation dans le corps humain. A l'intérieur de cette gaine 20, le fil conducteur 21 est formé d'un ou plusieurs brins enroulés en une spirale très serrée ce qui permet d'obtenir une sonde flexible afin d'être introduite dans les différents canaux veineux mais ne s'écrasant pas. Le diamètre des brins ainsi que le diamètre d'enroulement du conducteur 21 peut être variable d'un modèle de sonde à l'autre. De même, l'épaisseur de paroi de la gaine 20 peut être variable d'un modèle à l'autre. Après avoir coupé la sonde 2, on saisit le fil conducteur 21, au besoin après l'avoir dénudé, et on le tire à l'extérieur de la gaine 20 comme représenté à la figure 3. Le fil conducteur 21 est alors maintenu à l'extérieur par un pincement de ladite gaine 20 s'il a tendance à se rétracter.

On enfile alors la première partie 3 du connecteur sur l'extrémité de la sonde 2.

Cette première partie 3 du connecteur est formée d'un manchon arrière en matière plastique élastique et isolante solidaire d'une bague proximale rigide 32 qui en dépasse à une extrémité. La bague proximale 32 est enfoncée axialement à l'intérieur du manchon arrière 30 sur au moins une partie de sa longueur.

Le manchon arrière 30 est globalement cylindrique et est au diamètre préconisé par la norme pour la zone 16.

A son extrémité dont dépasse la bague proximale 32, le manchon 30 présente des anneaux 38 de dimensions et de position respectant la norme de la zone 14. La partie dépassante de la pièce 32 respecte quant à elle la géométrie déterminée pour la zone 12.

Le manchon arrière 30 comporte également une jupe souple et élastique 31 qui le recouvre.

La bague proximale 32 est globalement cylindrique ; un épaulement 33 sur sa surface externe permet son positionnement dans le manchon arrière 30.

Le manchon arrière 30 et la bague proximale 32 présentent des alésages 35, 34 en prolongement l'un de l'autre de diamètre supérieur au maximum des diamètres externes des conducteurs existants.

L'alésage 34 de la pièce 32 se raccorde par une portion conique 37 à un alésage 36 de plus grand diamètre qui se termine par une portion taraudée à l'extrémité libre de la pièce 32.

Cette première partie 3 du connecteur est enfilée sur le fil conducteur 21 de manière à ce que le manchon arrière 30 soit positionné contre la gaine 20, tandis que le fil conducteur 21 dépasse de l'extrémité libre de la bague proximale 32. Une fois la première partie 3 ainsi positionnée, l'on saisit la deuxième partie 4 du connecteur.

On peut à cet effet s'aider d'un outil tournevis 5 présentant un prolongement 51 de section hexagonale adapté à être introduit dans un logement 41 de forme femelle complémentaire de la seconde partie 4 du connecteur. Les dimensions de l'extrémité hexagonale seront avantageusement choisies de manière à être identiques aux dimensions couramment employées pour les systèmes de blocage des connecteurs des stimulateurs.

La seconde partie 4 est formée d'une fiche distale électriquement conductrice 42 entourée d'un manchon avant tubulaire 43 en matière plastique sur une partie de sa longueur.

La portion de la pièce 42 dépassant du manchon du côté présentant le logement 41 respecte les dimensions normalisées pour la zone conductrice 11 tandis que le manchon avant 43 est de dimension adaptée à former la zone 15 du conducteur normalisé et présente des anneaux 46 conformes à la zone 13.

La pièce 42 est globalement cylindrique avec un diamètre externe adapté à pénétrer dans l'alésage 36 de la première partie 3 du connecteur ; cette pièce 42 est filetée sur une longueur adaptée à coopérer avec le taraudage de l'alésage 36.

La pièce 42 présente un épaulement 44 pour le positionnement du manchon avant 43.

L'extrémité de la seconde partie 4 du connecteur destinée à s'engager dans l'alésage 36 de la première partie 3, porte un stylet 45.

Le stylet 45 est une pièce conductrice de diamètre externe inférieur au diamètre interne de l'enroulement du fil conducteur 21. Ce stylet 45 a globalement la forme d'un Z couché se terminant par des portions axiales. La distance séparant les deux portions radialement externes est supérieure au diamètre interne de l'alésage 34 de la première partie. Ce stylet peut avantageusement être terminé par une extrémité sphérique 47.

Comme on peut le voir aux figures 4 et 5, on enfile ledit stylet 45 dans l'extrémité dépassante du conducteur 21, l'enroulement dudit conducteur épousant grâce à sa flexibilité la forme externe du stylet.

La seconde partie 4 de l'adaptateur est peu à peu enfilée dans l'alésage 36 de la première partie 3.

Le conducteur 21 rigidifié en Z par le stylet 45 entre alors en contact par ses parties radialement externes avec la portion conique 37 de l'alésage de la première partie 3 ce qui l'oblige à s'aplatir pour pénétrer dans l'alésage 34 de plus petit diamètre.

Le stylet 45 étant en un matériau conducteur présentant une certaine élasticité coince alors le conducteur 21 entre lui-même et l'alésage de la pièce 32, ce gui assure une liaison électrique et mécanique entre le conducteur 21 et la fiche distale 42.

A cet effet, l'alésage 36 peut être strié annulairement de manière à augmenter le coefficient de frottement et améliorer ainsi la tenue mécanique du conducteur 21 dans le connecteur.

Lorsque la deuxième partie 4 est enfoncée, elle est solidarisée à la première partie par la collaboration du filetage de la pièce 42 avec le taraudage de la pièce 32 comme on peut le voir aux figures 6 et 7 jusqu'à ce que l'épaulement prévu sur la pièce 42 vienne en butée contre la pièce 32 ; le manchon avant 43 vient alors s'appuyer contre la pièce 32.

Le raccordement mécanique et électrique du conducteur 21 avec les deux parties 3, 4 du connecteur étant réalisé, on assure une liaison mécanique ainsi qu'une parfaite étanchéité de l'assemblage entre la gaine 20 de la sonde 2 et le connecteur en enduisant la gaine de colle 6 et en déroulant la jupe 31 qui vient recouvrir la partie enduite de la gaine 20 et étrangler la gaine 20 dans la zone 39 solidarisant ainsi ladite gaine 20 par serrage et collage comme représenté aux figures 7 et 8.

Au lieu d'une jupe 31 collée sur la gaine 20 de la sonde pour solidariser la sonde et le connecteur, on peut prévoir de coller directement la gaine 20 dans le connecteur 3. A cet effet, le manchon 30 est muni, vers l'arrière sur une partie de sa longueur, d'un alésage suffisant pour recevoir la gaine 20. Au moment de l'insertion de la gaine 20 dans le manchon 30, on injecte une colle pour assurer leur solidarisation.

On voit à la figure 9 le connecteur final ainsi obtenu, celui-ci présente les différentes zones 11, 12, 13, 14, 15, 16 définies par la norme qui sont formées par les pièces 30, 32, 42, 43.

Un connecteur universel selon l'invention permet, ainsi que l'on aura pu le comprendre, de remplacer de manière simple et sûre le connecteur d'une sonde monopolaire quelconque par un connecteur normalisé lorsque l'on change le stimulateur d'un patient.

## Revendications

1. Connecteur mâle universel de remplacement d'un ancien connecteur supprimé, de dimensions normalisées, destiné à être monté sur une sonde cardiaque monopolaire (2) formée par une gaine (20) dans laquelle est positionné un fil conducteur (21) enroulé en spirale, qui est formé par deux parties (3, 4) solidarisables l'une à l'autre,
- la première partie (3) étant formée d'un manchon arrière (30) en matière plastique présentant des anneaux (38) et traversé par un alésage (35) dans lequel peut coulisser le fil conducteur (21) de la sonde (2),
- la deuxième partie (4) étant formée d'une fiche distale électriquement conductrice (42)
- une bague proximale rigide (32) dépassant dudit manchon (30) du côté des anneaux (38), s'étendant dans la première partie (3), à l'intérieur du manchon arrière (30), sur au moins une partie de celui-ci,
- la fiche distale (42) portant dans la deuxième partie (4), un manchon avant (43) en matière plastique globalement au milieu de sa longueur ; ledit manchon avant (43) présentant des anneaux (46) et la fiche distale conductrice (42) dépassant dudit manchon (43) du côté des anneaux (46),
- la bague proximale (32) de la première partie (3) étant traversée par un alésage (34, 36, 37) dans lequel peut coulisser le fil conducteur (21) de la sonde (2),
caractérisé en ce que :
- un moyen conducteur (45) présentant une certaine élasticité et ayant globalement la forme d'un Z couché est prévu en combinaison avec la bague proximale (32) afin d'assurer une liaison électrique entre le fil conducteur (21) et la fiche distale (42) ainsi qu'une liaison mécanique entre le fil conducteur et le connecteur formé des premières (3) et deuxième partie (4), et en ce que l'alésage (36) est taraudé tandis que l'extrémité de la pièce conductrice (42) portant ledit moyen conducteur (45) est filetée.

2. Connecteur selon la revendication 1, caractérisé en ce que le moyen conducteur (45) présentant une certaine élasticité est un stylet (45) solidaire de la deuxième partie (4), formé par une pièce conductrice de diamètre externe inférieur au diamètre interne de l'enroulement du fil conducteur (21), ledit stylet se terminant par des portions axiales, la distance séparant les deux portions radialement externes étant supérieure au diamètre de l'alésage (34) de la bague proximale (32) de la première partie (3).

3. Connecteur selon l'une des revendications 1 ou 2, caractérisé en ce que l'alésage de la première partie (3) est formé d'une portion (35, 34) dans le manchon arrière (30) et dans la bague (32), de diamètre supérieur au maximum des diamètres externes des conducteurs (21) existants, raccordée par une portion conique (37) à un alésage (36) de plus grand diamètre.

4. Connecteur selon l'une quelconque des revendications précédentes, caractérisé en ce que le manchon arrière (30) est recouvert d'une jupe souple élastique (31) au cours du montage destinée à être collée sur la face externe de la gaine 20.

5. Connecteur selon la revendication 3, caractérisé en ce que l'alésage 36 prévu dans la bague proximale (32) peut être strié annulairement.

6. Connecteur selon la revendication 4, caractérisé en ce que la jupe (31) possède un étranglement (39) qui comprime la gaine (20).

## Claims

1. Universal male connector to replace a discarded old connector, of standardised dimensions, intended to be mounted on a monopolar cardiac stimulation probe (2) formed by a sheath (20) in which is positioned a conducting wire (21) wound into a spiral and formed by two portions (3, 4) that can be made integral with one another,
- the first portion (3) being formed of a rear sleeve (30) in plastic having rings (38) and being passed through by a bore (35) in which the conducting wire (21) of the probe (2) can be slid,
- the second portion (4) being formed of an electrically conductive distal plug (42),
- a rigid proximal ring (32) protruding from said sleeve (30) on the side of the rings (38), extending into the first portion (3), inside the rear sleeve (30) over at least a portion of the latter,
- the distal plug (42) bearing in the second portion (4) a front sleeve (43) in plastic globally in the middle of its length ; said front sleeve (43) having rings (46) and the conductive distal plug (42) protruding from said sleeve (43) on the side of the rings (46),
- the proximal ring (32) of the first portion (3) being passed through by a bore (34, 36, 37) in which the conducting wire (21) of the probe (2) can slide,
characterized in that:
- a conducting means (45), having a certain elasticity and being globally in the shape of a "Z" lying on its side, is provided in combination with the proximal ring (32) in order to establish an electrical connection between the conducting wire (21) and the distal plug (42), as well as a mechanical connection between the conducting wire and the connector formed by the first (3) and second (4) portions, and in that the bore (36) is tapped whereas the end of the conducting part (42) bearing said conducting means (45) is threaded.

2. Connector as claimed in claim 1, characterized in that the conducting means (45) having a certain elasticity is a stylet (45) integral with the second portion (4), formed by a conductive part of outside diameter smaller than the inside diameter of the winding of conducting wire (21), said stylet ending in axial portions, the distance separating the two radially external portions being greater than the diameter of the bore (34) of the proximal ring (32) of the first portion (3).

3. Connector as claimed in either claim 1 or claim 2, characterized in that the bore of the first portion (3) is formed of a portion (35, 34), in the rear sleeve (30) and in the ring (32), of diameter greater than the maximum of the outside diameters of the existing conductors (21), connected by a conical portion (37) to a bore (36) of larger diameter.

4. Connector as claimed in any one of the previous claims, characterized in that, during assembly, the rear sleeve (30) is clad in a flexible elastic skirt (31) destined to be bonded to the outer face of the sheath 20.

5. Connector as claimed in claim 3, characterized in that the bore 36 provided in the proximal ring (32) can have annular striations.

6. Connector as claimed in claim 4, characterized in that the skirt (31) has a contraction in area (39) which compresses the sheath (20).

## Patentansprüche

1. Universaler Steckverbinder zum Austausch von einem früheren, weggenommenen Steckverbinder, mit Standarddimensionen, der für Zusammenbauen mit einer monopolaren Herzreizungssonde (2) vorgesehen ist, die von einer Umhüllung (20) und einem in dieser angebrachten spiralförmigen Leitungsdraht (21) ausgeführt ist, wobei der Steckverbinder von zwei miteinander fest zu verbindenden Bauteilen gebildet ist und
- das erste Bauteil (3) von einer mit Ringen (38) ausgebildeten und von einer durch eine Bohrung durchquerten hinteren Muffe (30) aus Kunststoff besteht,
- das zweite Bauteil (4) von einem distalen, elektrisch leitenden Stöpsel (42) besteht,
- eine steife, proximale, aus der Muffe in der Nähe der Ringen (38) hervorragende Hülse (32) sich im ersten Bauteil (3), im Innere der hinteren Muffe auf mindestens ein Teil davon, erstreckt,
- in das zweite Bauteil (4) der distale Stöpsel (42) ungefähr in der Mitte von seiner Länge eine vordere Muffe (43) aus Kunststoff trägt; wobei die vordere Muffe Ringen (46) aufweist und der elektrisch leitende Stöpsel (42) aus der Muffe (43) in der Nähe der Ringen (46) hervorragt,
- die proximale Hülse (32) des ersten Bauteils (3) von einer Bohrung (34, 36, 37) durchquert ist, in welcher der Leitungsdraht (21) der Sonde (2) gleiten kann,
dadurch gekennzeichnet, daß :
- ein eine gewisse Elastizität und eine Form von ungefähr einem liegenden Z aufweisendes, mit der proximale Hülse (32) kombiniertes, leitendes Mittel (45) angeordnet ist, um eine elektrische Verbindung zwischen dem Leitungsdraht (21) und dem distalen Stöpsel (42), sowie eine mechanische Verbindung zwischen dem Leitungsdraht und dem von den ersten und zweiten Bauteilen gebildeten Steckverbinder zu bewirken, und die Bohrung (36) ein Gewinde aufweist und das Ende des das leitende Mittel tragenden leitenden Teils hingegen ein Außengewinde aufweist.

2. Steckverbinder nach Anspruch 1, dadurch gekennzeichnet, daß das eine gewisse Elastizität aufweisende leitende Mittel (45) ein mit dem zweiten Bauteil (4) fest verbundenes Stäbchen ist das einen äußeren Durchmesser der kleiner als der innere Durchmesser der Wicklung des Leitungsdrahts (21) aufweist, wobei das Stäbchen in axialen Abschnitten endet und der Abstand zwischen den zwei radial äußeren Abschnitten größer als der Durchmesser der Bohrung (34) in der proximale Hülse (32) des ersten Bauteils ist.

3. Steckverbinder nach Anspruch 1 oder 2, dadurch gekennzeichnet, daß die Bohrung des ersten Bauteils (3) von einem Abschnitt (35, 34) in der hinteren Muffe (30) und in der Hülse (32) gebildet ist, wobei der Durchmesser dieses Abschnitts größer als die maximale Durchschnitte der vorhandenen Leiter (21) ist, und der Abschnitt mittels eines kegelförmigen Abschnitts (37) mit einer Bohrung (36) von größerem Durchmesser verbunden ist.

4. Steckverbinder nach einem der vorhergehenden Ansprüche, dadurch gekennzeichnet, daß die hintere Muffe (30) während des Zusammenbaus durch einen elastischen nachgiebigen Mantel überdeckt ist, wobei dieser Mantel für das Ankleben an die Außenseite der Umhüllung (20) vorgesehen ist.

5. Steckverbinder nach Anspruch 3, dadurch gekennzeichnet, daß die in der proximale Hülse (32) angeordnete Bohrung (36) ringförmig gerillt ausgeführt werden kann.

6. Steckverbinder nach Anspruch 4, dadurch gekennzeichnet, daß der Mantel (31) eine die Umhüllung (20) zusammendrückende Einschnürung (39) aufweist.
